(19) Europäisches Patentamt
European Patent Office
Office européen des brevets

(11) Publication number: **0 467 339 A1**

(12) **EUROPEAN PATENT APPLICATION**

(21) Application number: **91111963.4**

(22) Date of filing: **17.07.91**

(51) Int. Cl.5: **C08F 220/32**, //C08F2/18, C08F8/00,B01J20/26, B01J39/20,B01J41/14, B01D15/08,(C08F220/32, 220:18)

(30) Priority: **18.07.90 JP 188003/90**
**07.08.90 JP 207503/90**
**09.08.90 JP 209296/90**

(43) Date of publication of application:
**22.01.92 Bulletin 92/04**

(84) Designated Contracting States:
**DE FR GB**

(71) Applicant: **MITSUBISHI KASEI CORPORATION**
**5-2, Marunouchi 2-chome Chiyoda-ku**
**Tokyo 100(JP)**

(72) Inventor: **Ito, Tsuyoshi**
**1-8-20, Ogawa**
**Machida-shi, Tokyo(JP)**
Inventor: **Takayanagi, Hiroaki**
**2-8-2, Ogawa**
**Machida-shi, Tokyo(JP)**

(74) Representative: **Wächtershäuser, Günter, Dr.**
**Tal 29**
**W-8000 München 2(DE)**

(54) **Water-swellable crosslinked polymer particles and process for their production.**

(57) A hydrolyzate of a crosslinked copolymer of glycidyl methacrylate with a crosslinking agent of the formula (1):

$$CH_2=C(CH_3)-COO(R-O)_nOC-C(CH_3)=CH_2 \qquad (1)$$

wherein R is a $C_{2-4}$ alkylene group, and n is an integer of from 1 to 4, which has an apparent volume in a dried state of from 2.0 to 3.0 mℓ/g and an apparent volume in a state swelled with water of from 6.0 to 13.0 mℓ/g.

EP 0 467 339 A1

The present invention relates to crosslinked polymer particles remarkably swellable in water and a process for their production.

It is common to recover a certain specific macromolecular substance from an aqueous solution containing various macromolecular substances. As an example, a protein is recovered from an aqueous solution. For example, it is common to recover an enzyme or a physiologically active protein from a culture medium of microorganisms or cells. It is also practiced to recover a specific protein such as albumin from a blood. Further, it is also practiced to recover a protein from whey. Several methods are practically used for the recovery of a macromolecular substance such as a protein from an aqueous solution. Among them, there is a method wherein a molecular sieve or adsorbent (which will be hereinafter generally referred to as a separating agent) is employed. For example, it is known to separate and recover a specific protein contained in an aqueous solution in admixture with other proteins by passing the solution through a column packed with gel particles produced by reacting a crosslinking agent to a polysaccharide such as cellulose or dextran and which is capable of swelling in water without being dissolved. This method is referred to as a gel filtration chromatography, which utilizes a molecular sieving effect of the gel particles i.e. a phenomenon such that the time required for solute molecules to diffuse in the gel particles varies depending upon the size of the solute molecules in the aqueous solution. By such gel filtration chromatography, it is possible to separate and recover a desired protein from an aqueous solution containing a plurality of proteins by adjusting the network size of gel particles. To separate and recover an ionizable macromolecular substance, it is known to employ, as the separating agent, gel particles having ion exchange groups in the interior. For example, when an aqueous alkaline solution containing a macromolecular substance having carboxyl groups and a nonionic macromolecular substance, is passed through a column packed with gel particles having tertiary amino groups in the interior, the macromolecular substance having carboxyl groups diffuses into the gel particles and will be captured by the amino groups present in the interior, whereas, the nonionic macromolecular substance will not be captured even when diffused into the gel particles and will pass through the particles. After the nonionic macromolecular substance has flowed away from the column, an eluent is passed through the column, whereby the macromolecular substance having carboxyl groups, which has been captured in the gel particles, will be eluted and can be recovered. To separate such an ionizable macromolecular substance, it is possible to employ, as the separating agent, a material obtained by introducing ion exchange groups such as amino groups or sulfonic acid groups to the above-mentioned gel particles obtained by reacting a crosslinking agent to a polysaccharide from a natural source. The gel particles prepared from such a natural material, are usually poor in the physical strength and have drawbacks such that they are susceptible to decomposition with an acid or alkali and they are susceptible to degradation by microorganisms.

It has been proposed to employ particles of a hydrophilic synthetic polymer instead of such gel particles made of natural material. For example, in Japanese Examined Patent Publication No. 58026/1983, it is disclosed to conduct chromatography by using, as the separating agent, hydrophilic porous crosslinked copolymer particles having pendent glycerol residues, prepared by suspension-polymerizing a mixture of glycidyl methacrylate and ethylene glycol dimethacrylate in an aqueous medium to obtain porous crosslinked copolymer particles having glycidyl groups, followed by hydrolyzing the glycidyl groups to glycerol groups. This method is referred to as gel permeation chromatography, because as is different from the above-mentioned separating agent made of a macromolecular material from a natural source, such porous crosslinked copolymer particles have numerous pores in the interior, whereby the solute molecules diffuse into such pores as opposed to the space of the network of the particles. Accordingly, the research and development for such a porous separating agent has been generally directed to control the size of pores and to enlarge the total volume of pores which contribute to the diffusion of the solute as far as possible. Further, in Japanese Unexamined patent Publication No. 90991/1978, it is disclosed to obtain a porous separating agent having amino groups by reacting e.g. dimethylamine to porous crosslinked copolymer particles having glycidyl groups. The substantial difference between such a porous separating agent made of a synthetic polymer and the above-mentioned separating agent made of a natural material is that the former does not or scarcely swell in water, while the latter remarkably swells in water. The particles of the former have a dense, rigid skeleton made of a crosslinked copolymer and a space surrounded by this skeleton i.e. pores, and thus a very large apparent volume (mℓ/g) in its dried state. When such particles are put in water, water penetrates into the space in the particles, but the rigid skeleton prevents swelling of the particles. Whereas, the latter hydrophilic separating agent made of a natural material does not have pores or rigid skeleton in its dried state. When such a separating agent is put into water, water penetrates into the network of the particles to expand the network, whereby the particles undergo swelling. Usually, the lower the density of the crosslinkage, the higher the degree of the swelling.

The present invention is intended to provide a separating agent made of a synthetic polymer which is

capable of remarkably swelling in water i.e. which is similar to the above-mentioned separating agent made of a natural material.

It is one object of the present invention to provide a separating agent having pendent glycerol residues, which is capable of remarkably swelling in water and which is accordingly suitable for gel filtration chromatography.

Another object of the present invention is to provide a separating agent having pendent glycerol residues and sulfonic acid groups or amino groups, which is capable of remarkably swelling in water and which is accordingly suitable for separation of an ionizable macromolecular compound by adsorption.

A further object of the present invention is to provide a method for separating a macromolecular compound such as a protein by chromatography in which such a separating agent is employed.

A still further object of the present invention is to provide a method for separating a protein by adsorption from an aqueous solution by means of the above-mentioned separating agent having sulfonic acid groups or amino groups.

Another object of the present invention is to provide a process for the production of such a separating agent.

A further object of the present invention is to provide a crosslinked copolymer having glycidyl groups, which is suitable for the preparation of such a separating agent, and a process for its production.

The separating agent of the present invention is particles of a crosslinked polymer having pendent glycerol residues. The polymer may further have amino groups or sulfonic acid groups. It has a relatively small apparent volume of from 2.0 to 3.0 mℓ/g in a dried state. However, it has a characteristic such that in water, it absorbs water and swells to a volume of at least twice, usually at least three times, the volume in the dried state.

To produce the separating agent of the present invention, glycidyl methacrylate and a dimethacrylate of the formula (1) as a crosslinking agent, are copolymerized to obtain a crosslinked copolymer having glycidyl groups:

$$CH_2=C(CH_3)-COO\text{-}(R-O)_n\text{-}OC-C(CH_3)=CH_2 \qquad (1)$$

wherein R is a $C_{2-4}$ alkylene group such as ethylene, n-propylene or i-propylene, and n is an integer of from 1 to 4.

As the crosslinking agent, preferred is a dimethacrylate of ethylene glycol or a dimethacrylate of ethylene glycol oligomer represented by the following formula (2):

$$CH_2=C(CH_3)-COO\text{-}(CH_2-CH_2-O)_n\text{-}OC-C(CH_3)=CH_2 \qquad (2)$$

The separating agent which is made of a copolymer of glycidyl methacrylate with a crosslinking agent of the formula (2), is usually rich in swellability in water and poor in interaction with protein, thus suitable as a separating agent for a protein.

Particularly preferred as a crosslinking agent is diethylene glycol dimethacrylate, because it has a reactivity similar to glycidyl methacrylate, whereby it is possible to avoid an undesirable phenomenon such that only one of the monomers reacts preferentially in the course of copolymerization, and the composition of the resulting copolymer varies as between the initial stage and the last stage of the reaction.

The proportion of the crosslinking agent in the monomer mixture subjected to the copolymerization reaction is in the range of from 3 to 15% based on the vinyl group. Usually the proportion is in the range of from 2.5 to 10% by weight. If the proportion of the crosslinking agent increases, a porous crosslinked copolymer tends to form. At the same time, the swellability in 1,4-dioxane of the resulting crosslinked copolymer and the swellability in water of a separating agent prepared from such a crosslinked copolymer tend to be low. On the other hand, if the proportion of the crosslinking agent in the monomer mixture decreases, the swellability of the product usually increases, but the physical strength of the separating agent finally obtained, tends to be low. The proportion of the crosslinking agent in the monomer mixture is preferably from 3 to 10% by weight, more preferably from 4 to 9% by weight.

The copolymerization reaction can be conducted by various conventional methods. It is usual, however, to employ a method of suspension-polymerizing the monomer mixture in an aqueous medium in the presence of a dispersion stabilizer and an inorganic salt. As the dispersion stabilizer, gellatin, sodium

polyacrylate, carboxymethylcellulose, polyvinyl alcohol or the like may be employed. As the inorganic salt, sodium chloride, calcium chloride, sodium sulfate or the like may be employed. Such an inorganic salt dissolved in the aqueous medium serves to prevent the monomers form dissolving into the aqueous medium.

The suspension polymerization is conducted by suspending in the above aqueous medium an organic solution comprising glycidyl methacrylate, the crosslinking agent, a polymerization initiator and an organic solvent which is capable of dissolving such components and which is not soluble in the aqueous medium, and reacting the suspension at a temperature of from 50 to 90°C for from 6 to 20 hours. It is, of course, possible to conduct the reaction at a lower temperature for a longer period of time, if desired. As the polymerization initiator, conventional initiators including organic peroxides such as benzoyl peroxide and t-butyl hydroperoxide, and organic azobis compounds such as azobisisobutyronitrile and 2,2'-azobis(2,4-dimethylvaleronitrile), can be used. The polymerization initiator is used usually in an amount of from 0.01 to 5% by weight relative to the monomer mixture.

As the organic solvent, toluene, cyclohexanol, l-octanol, dibutyl ether, n-butyl acetate or isooctane may, for example, be used. The organic solvent is used usually in an amount of from 0.5 to 1.5 times by volume at room temperature relative to the monomer mixture. When the monomer mixture is dissolved in an organic solvent and subjected to suspension polymerization, spherical crosslinked copolymer particles can readily be produced. As the proportion of the organic solvent to the monomer mixture increases, the apparent volume of the resulting crosslinked copolymer in a dried state usually tends to be large. This tendency is remarkable especially in the case of a so-called poor solvent to a homopolymer of glycidyl methacrylate, such as isooctane or l-octanol. Further, if such a poor solvent is used, the resulting crosslinked copolymer tends to be porous. Accordingly, when the proportion of the crosslinking agent in the monomer mixture is large, it is preferred to employ e.g. toluene or cyclohexanol instead of such a poor solvent, thereby to avoid or reduce formation of pores in the resulting crosslinked copolymer.

In the suspension polymerization, the ratio (weight ratio) of the organic phase to the aqueous medium phase is preferably adjusted to a level within a range of from 1:3 to 1:10. If the aqueous medium phase is too much, the amount of the monomers dissolving in the aqueous medium phase increases, and the yield of the crosslinked copolymer particles decreases. On the other hand, if the aqueous medium phase is too little, the suspension tends to be unstable, and it becomes difficult to obtain spherical crosslinked copolymer particles.

The resulting crosslinked copolymer particles having glycidyl groups preferably have the following properties so that they can be readily led to a separating agent of the present invention.

Apparent volume in a dried state:
1.8-3.0 mℓ/g, especially 2.0-3.0 mℓ/g
Apparent volume in water:
1.8-3.0 mℓ/g, especially 2.0-3.0 mℓ/g
Apparent volume in 1,4-dioxane:
5.0-15.0 mℓ/g

Namely, it is preferred that the particles of the crosslinked copolymer having glycidyl groups do not substantially swell in water and remarkably swell in 1,4-dioxane. The ratio of the apparent volume in 1,4-dioxane to the apparent volume in a dried state should preferably be at least 2.5.

To increase the apparent volume in a dried state and in water, the ratio of the organic solvent to the monomer mixture may be increased for the suspension polymerization, or a poor solvent may be employed as the organic solvent. To increase the apparent volume in dioxane, the proportion of the crosslinking agent in the monomer mixture may be made small.

The particle size of the crosslinked copolymer having glycidyl groups is usually from 5 to 2000 μm, but specifically, the particle size is determined depending upon the desired particle size of the separating agent to be finally obtained. For example, in a case where a separating agent having pendent glycerol residues useful for gel filtration chromatography, is to be obtained, the particle size of the crosslinked copolymer having glycidyl groups is adjusted relatively small, usually to a level of from 10 to 150 μm, preferably from 30 to 100 μm, in a dried state to get a better result. In a case where a separating agent having amino groups or sulfonic acid groups in addition to pendent glycerol residues is to be obtained, the particle size of the crosslinked copolymer having glycidyl groups is usually from 70 to 300 μm, in a dried state because the larger the particle size of the separating agent, the easier the handling.

When the above crosslinked copolymer having glycidyl groups is reacted with water to have the glycidyl groups hydrolyzed to glycerol groups, a separating agent having pendent glycerol residues of the present invention can be obtained. This hydrolysis is preferably conducted in such a manner that the above crosslinked copolymer having glycidyl groups is swelled in a hydrophilic organic solvent, and water and an

acid as a catalyst, are added to this swelled crosslinked copolymer for reaction, whereby it is possible to obtain a separating agent which has the following apparent volumes and which is thus capable of remarkably swelling in water:

| | |
|---|---|
| Apparent volume in a dried state: | 2.0-3.0 mℓ/g |
| Apparent volume in water: | 6.0-13.0 mℓ/g |

Whereas, when the aqueous acid solution is added without having the crosslinked copolymer having glycidyl groups preliminarily swelled with an organic solvent, and the mixture is heated, the glycidyl groups can be hydrolyzed to form glycerol residues, but the resulting separating agent tends to be poor in the swellability in water.

As the organic solvent useful for swelling the crosslinked copolymer having glycidyl groups, 1,4-dioxane is usually employed. It is also possible to employ a hydrophilic organic solvent such as tetrahydrofuran, N,N-dimethylformamide or dimethylsulfoxide, which is capable of being dissolved at an optional proportion in water and which is capable of swelling the crosslinked copolymer particles to a volume of at least two times. When the crosslinked copolymer having glycidyl groups is put in such an organic solvent and maintained at room temperature or under heating for from 1 to 5 hours, it swells usually at least twice by volume. It is usually preferred to let it swell until no volume increase will be seen any longer. Then, while the swelled crosslinked copolymer is suspended in a large amount of an organic solvent, an aqueous acid solution is added thereto in a large excess relative to the glycidyl groups, and the mixture is held at a temperature of from 20 to 90 °C for from 0.5 to 5 hours, whereby the glycidyl groups will be converted to glycerol residues. It is possible to confirm that the glycidyl groups have been completely hydrolyzed by observing that there is no absorption at about 900 $cm^{-1}$ corresponding to the absorption by an epoxy ring in the infrared absorption spectrum of the particles subjected to the hydrolysis.

The hydrolysis is preferably conducted under a mild condition to avoid cleavage of ester bonds in the crosslinked copolymer. The carboxyl groups formed by the cleavage of the ester bonds will give an adverse effect to the separation of a macromolecular compound having ionic functional groups, such as a protein. The amount of carboxyl groups in a separating agent having glycerol residues obtained by the hydrolysis, is preferably not more than 0.1 meq/mℓ, more preferably not more than 0.05 meq/mℓ, in a state swelled in water.

The water-swellable polymer particles having pendent glycerol residues thus obtained can be used by themselves as a separating agent for gel filtration chromatography. In such a case, the particle size is usually from 20 to 300 µm, preferably from 50 to 150 µm in the state swelled in water.

The separating agent of the present invention may have, in addition to the pendent glycerol residues, tertiary amino groups or sulfonic acid groups. Such a water swellable separating agent having amino groups or sulfonic acid groups, is useful particularly for the separation and recovery of a macromolecular compound having amino groups or carboxyl groups such as certain types of proteins. Among such separating agents having amino groups or sulfonic acid groups, a particularly preferred is the one which is capable of remarkably swelling in water i.e. which has the following properties:

| | |
|---|---|
| Apparent volume in a dried state: | 2.0-3.0 mℓ/g |
| Apparent volume in water: | 6.0-13.0 mℓ/g |

The ion exchange capacity of such a separating agent is usually from 0.05 to 0.5 meq, preferably not higher than 0.4 meq, per mℓ of the resin in the state swelled in water.

A protein usually has a very large amino group equivalent and carboxyl group equivalent (which corresponds to the molecular weight per mol of the amino group or the carboxyl group). Accordingly, as is different from a case of capturing an ionized compound having a low molecular weight, in a case of capturing a protein, the separating agent may be the one having a relatively small ion exchange capacity per unit volume. If the ion exchange capacity is large, there will be a disadvantage such that a large amount of an eluent will be required to elute the adsorbed protein.

When the separating agent of the present invention is used for the separation and recovery of a protein by ion exchange or ion exchange chromatography, the ion exchange capacity of the separating agent is preferably from 0.1 to 0.3 meq per mℓ of the resin in the state swelled with water. Further, in a case where the ion exchange function of the separating agent is utilized, the particle size of the separating agent is preferably relatively large. Accordingly, the separating agent of the present invention having amino groups

or sulfonic acid groups preferably has a particle size within a range of from 100 to 500 $\mu$m in the state swelled in water.

To obtain a separating agent having pendent glycerol residues and amino groups and capable of remarkably swelling in water, amino groups may be introduced into the above-mentioned water-swellable polymer particles having pendent glycerol residues.

The introduction of amino groups is preferably conducted in two stages by putting the above-mentioned water-swellable polymer particles having pendent glycerol residues into a solution mixture comprising an aqueous alkali metal hydroxide solution and a hydrophilic organic solvent to let the solution mixture penetrate into the polymer particles so that the polymer particles will be swelled with the solution mixture and at the same time, the hydroxyl groups of the glycerol residues will be activated by the alkali metal hydroxide, and then adding an amine having a haloalkyl group or its salt for the reaction while maintaining the swelled state. It is thereby possible to obtain a basic anion exchange resin having substantially the same apparent volume in a dried state as the starting material polymer particles having pendent glycerol residues and more swellable in water.

Whereas, if the polymer particles having pendent glycerol residues are put into an aqueous alkali metal hydroxide solution and then an amine having a haloalkyl group or its salt is added thereto, the particles tend to shrink during the reaction, and it is thereby hardly possible to obtain a basic anion exchange resin well swellable in water.

As the hydrophilic organic solvent, it is common to employ a monohydric or polyhydric alcohol. A $C_{2-4}$ alcohol is preferred since it has good compatibility with an aqueous alkali metal hydroxide solution. Among them, an alcohol having a secondary hydroxyl group, particularly isopropanol, is preferred, since an aqueous isopropanol solution containing an alkali metal hydroxide is considered to be particularly effective for activation of the hydroxyl groups of the glycerol residues. The hydrophilic organic solvent is preferably used in an amount of from 0.3 to 3 times by volume to the aqueous alkali metal hydroxide solution. The hydrophilic organic solvent is believed to serve to maintain the swelled state of the polymer particles during the reaction of the polymer particles with the haloalkylamine and to promote the uniform diffusion of the haloalkylamine into the polymer particles.

As the alkali metal hydroxide, sodium hydroxide is usually employed. It is also possible to employ potassium hydroxide or lithium hydroxide. The concentration of the aqueous alkali metal hydroxide solution is preferably from 1 to 10 N. If the concentration of the aqueous solution is low, the action to activate the hydroxyl groups of the glycerol residues tends to be weak. On the other hand, if the concentration of the aqueous solution is too high, it is likely that cleavage of the ester bonds of the polymer takes place.

The solution mixture comprising the aqueous alkali hydroxide solution and the hydrophilic organic solvent, is preferably employed in a large excess to the polymer particles so that even after the swelling, the polymer particles can be present in the form of a slurry.

As the amine having a haloalkyl group, a tertiary amine of the formula (3) may be employed:

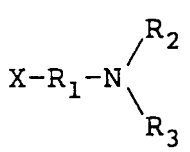

(3)

wherein X is chlorine, bromine or iodine, $R_1$ is an alkylene group, and each of $R_2$ and $R_3$ is an alkyl group or a hydroxyalkyl group.

In the formula (3), preferred as the haloalkyl group (X-$R_1$-) is a $C_{2-4}$ lower haloalkyl group such as a 2-chloroethyl group, a 2-bromoethyl group, a 3-chloropropyl group or a 4-chlorobutyl group. Further, preferred as each of $R_2$ and $R_3$ is a $C_{1-4}$ lower alkyl group or $C_{2-4}$ hydroxy lower alkyl group, such as a methyl group, an ethyl group, a 2-hydroxyethyl group, a n-propyl group, a n-butyl group or an i-butyl group. Particularly preferred is a 1-halo-2-(N,N-diethylamino)ethane or a 1-halo-2-(N,N-dimethylamino)ethane.

The amine is used usually in an amount of from 0.1 to 3 mol times to the glycerol residues of the polymer particles. The amine may be used in a free form or may be used in its salt such as a hydrochloride, a carbonate or a bicarbonate.

Namely, the polymer particles having glycerol residues are put into the above-mentioned solution mixture comprising the aqueous alkali metal hydroxide solution and the hydrophilic organic solvent, then the mixture is maintained at a temperature of from 20 to 90 °C for from 1 to 5 hours in the form of a slurry to activate the hydroxyl groups of the glycerol residues and to let the particles swell to a volume of at least twice the volume in the dried state, and then the above-mentioned tertiary amine is added thereto, and the

mixture is maintained for further 1 to 10 hours at a temperature of from 20 to 90 °C, whereby the haloalkylamine is reacted to the glycerol residues to form a basic anion exchange resin of the present invention. The separating agent having an anion exchange ability thus obtained, has substantially the same apparent volume in the dried state and the same or larger apparent volume in water as the polymer particles having pendent glycerol groups, used as the starting material.

A separating agent which has pendent glycerol residues and sulfonic acid groups and which is capable of remarkably swelling in water, can be obtained by reacting a sulfite or a hydrogensulfite to the above-mentioned swellable crosslinked copolymer having glycidyl groups, then permitting polymer particles to swell with a hydrophilic organic solvent, and then an aqueous acid solution is added thereto to hydrolyze the glycidyl groups to glycerol groups.

To introduce sulfonic acid groups to the swellable crosslinked copolymer having glycidyl groups, a sulfite or a hydrogensulfite may be reacted to such a copolymer in water. Usually, sodium sulfite or sodium hydrogensulfite is employed. To an aqueous solution of such a salt, wherein the concentration of the salt is from 0.1 mol/ℓ to saturation, the polymer is added, and the mixture is reacted at a temperature of from 30 to 90 °C for from 1 to 10 hours, whereby the swellable polymer having glycidyl groups will be converted to an acidic cation exchange resin having glycidyl groups and sulfonic acid groups. Then, the unreacted glycidyl groups are hydrolyzed to pendent glycerol residues. The hydrolysis of the glycidyl groups of the sulfonated polymer is required to be conducted in such a state that the polymer swells with a hydrophilic organic solvent, in the same manner as the hydrolysis of glycidyl groups of a crosslinked copolymer. If the polymer is merely put in an aqueous mineral acid solution to conduct the hydrolysis of glycidyl groups, it is hardly possible to obtain a product excellent in the swellability in water.

For the hydrolysis of the glycidyl groups of the sulfonated polymer, the polymer is introduced into a hydrophilic organic solvent soluble in water at an optional proportion, such as 1,4-dioxane, tetrahydrofuran, N,N-dimethylformamide or dimethylsulfoxide, and the mixture is maintained at room temperature for from 1 to 5 hours to let the polymer particles swell to a volume of at least twice the volume in the dried state. Then, an aqueous acid solution is added thereto, and the mixture is maintained at a temperature of from 20 to 90 °C for from 0.5 to 5 hours, whereby the glycidyl groups will be hydrolyzed to obtain a water-swellable acidic cation exchange resin having pendent glycerol residues and sulfonic acid groups.

The separating agent of the present invention is useful as a packing material for column chromatography. For example, an aqueous solution containing various proteins having different molecular weights, is supplied from the top of a column packed with the separating agent of the present invention, and permitted to flow slowly through the column, then water is supplied from the top of the column to discharge the aqueous solution, whereby the respective proteins in the aqueous solution will mutually be separated and flow out from the bottom of the column. The separating agent of the present invention has higher mechanical strength than the one made of a natural material, and it is thereby possible to increase the height of the packed column. For example, the separating agent of the present invention can be packed in a column of 50 cm or higher and used for chromatography.

Among the separating agents of the present invention, the one having amino groups or sulfonic acid groups is useful as an ion exchange resin. For example, an aqueous solution containing various proteins may be supplied to the top of a column packed with a separating agent having amino groups of the present invention and permitted to flow in the column. At that time, the pH of the aqueous solution is adjusted to a pH higher than the isoelectric point of the protein to be captured. The effluent from the bottom of the column is monitored, and when the protein to be captured has started to leak in the effluent, the supply of the aqueous solution to the column, is stopped, and instead, water is supplied to discharge the solution remaining in the column. Then, an aqueous acid solution having a low concentration, e.g. an aqueous hydrochloric acid solution of 0.1 mol/ℓ, is supplied to the column, whereupon the protein adsorbed on the separating agent will elute. Instead of the aqueous acid solution, it is possible to employ an aqueous sodium chloride solution having a concentration of from about 0.5 to 1 mol/ℓ.

Further, as an alternative method, the separating agent having an ion exchange ability of the present invention is added to an aqueous solution to adsorb a desired component thereon. Then, the separating agent is recovered by filtration or centrifugal separation, and the recovered separating agent is put into an aqueous acid solution or an aqueous sodium chloride solution to elute the adsorbed component. For example, to whey having the pH adjusted to an acidic level, the separating agent having sulfonic acid groups of the present invention is added to have lactalbumin and lactoglobulin in the whey adsorbed thereon. Then, the separating agent is put into an aqueous alkaline solution or in an aqueous sodium chloride solution of from about 0.5 to 1 mol/ℓ, whereby adsorbed proteins will elute. The separating agent of the present invention swells remarkably in water to permit diffusion of the protein into the interior of the separating agent, and thus the adsorption capacity to a protein is very large. For example, according to the

present invention, it is possible to have a protein adsorbed on a separating agent in an amount of at least 30 g, preferably at least 50 g per liter of the separating agent packed in the column.

The following methods were used for measuring various characteristics of the polymer particles.

Method for measuring the apparent volume of water-swellable polymer particles having glycerol residues.

About 10 mℓ of polymer particles swelled in water are put together with water into a 10 mℓ measuring cylinder, and the volume ($V_1$ mℓ) is measured. Then, the polymer particles are transferred to a beaker, and water is removed by decantation. Then, 100 mℓ of acetone is added thereto, and the mixture is maintained for 10 minutes. Then, acetone is removed by decantation, and 100 mℓ of acetone is added afresh and the mixture is maintained for 10 minutes. This operation is repeated once more. Then, acetone is removed by decantation, and 100 mℓ of toluene is added thereto, and the mixture is maintained for 10 minutes. Then, toluene is removed by decantation, and 100 mℓ of toluene is added afresh, and the mixture is maintained for 10 minutes. This operation is repeated once more. Toluene is removed by decantation, 100 mℓ of n-heptane is added thereto, and the mixture is maintained for 10 minutes. n-Heptane is removed by decantation, 100 mℓ of n-heptane is added afresh, and the mixture is maintained for 10 minutes. This operation is repeated once more. The polymer particles are recovered by filtration and dried under reduced pressure to evaporate n-heptane. The weight (Wg) of the dried resin is measured. Further, the dried resin is put into a 10 mℓ measuring cylinder, and the volume ($V_2$ mℓ) is measured.

| | |
|---|---|
| Apparent volume in a dried state of the polymer particles: | $V_2/W$ (mℓ/g) |
| Apparent volume of the polymer particles in a state swelled in water: | $V_1/W$ (mℓ/g) |

In the case of polymer particles having amino groups or sulfonic acid groups, the above operation is conducted after the amino groups or sulfonic acid groups are freed.

Method for measuring the ion exchange capacity of polymer particles having glycerol residues and amino groups

Polymer particles are treated with a 0.1N NaOH aqueous solution to covert the amino groups in free forms and then thoroughly washed with water. About 5 mℓ of the polymer particles are put into a 10 mℓ measuring cylinder, and the volume (V mℓ) is measured. After removing water by centrifugal separation, the polymer particles are transferred to a beaker. 100 mℓ of 0.1N HCℓ is added thereto and the mixture is maintained for 12 hours. Then, 10 mℓ of the supernatant is taken and titrated with a 0.1N NaOH aqueous solution. When the consumption of the 0.1N NaOH aqueous solution is Mmℓ, the ion exchange capacity of the polymer particles will be given by (10-M)/V meq/mℓ.

Method for measuring the ion exchange capacity of polymer particles having glycerol residues, or glycerol residues and sulfonic acid groups

Polymer particles are treated with a 0.1N HCℓ aqueous solution to covert the carboxyl groups into free forms, or the carboxyl groups and the sulfonic acid groups and then washed with water. About 5 mℓ of the polymer particles are put into a 10 mℓ measuring cylinder, and the volume (V mℓ) is measured. After removing water by centrifugal separation, the polymer particles are transferred to a beaker. 100 mℓ of a 0.1N NaOH solution is added thereto, and the mixture is maintained for 12 hours. Then, 10 mℓ of the supernatant is taken and titrated with a 0.1N HCℓ aqueous solution. When the consumption of the 0.1N HCℓ aqueous solution is Mmℓ, the ion exchange capacity of the polymer particles is given by (10-M)/V meq/mℓ.

Method for measuring sulfonic acid groups in the polymer particles having glycerol residues and sulfonic acid groups

Polymer particles are treated with a 0.1N HCℓ aqueous solution to covert sulfonic acid groups into free

forms and then thoroughly washed with water. About 5 mℓ of the polymer particles are put into a 10 mℓ measuring cylinder, and the volume (V mℓ) is measured. Then, the particles are dried in a vacuum dryer at 100 °C for 24 hours. The dried polymer particles are subjected to elemental analysis according to a conventional method to measure the content (W mg) of sulfur.

The amount of sulfonic acid groups in the polymer particles will be given by (W/32)/V meq/mℓ.

Method for measuring the exclusion limiting molecular weight

Into a glass column having an inner diameter of 8.2 mm, polymer particles having pendent glycerol residues are packed (height of packing: 47.5 cm). To this column, dextran is injected while continuously supplying deionized water at a rate of 0.5 mℓ/min, and the time until the dextran is detected in the effluent from the column, is measured.

The above measurement is repeated with dextrans having different molecular weights. From the results of the measurement, the exclusion limiting molecular weights of the dextrans are determined in accordance with a conventional method.

Method for measuring the absorption capacity for albumin

Polymer particles are put in a buffer solution (a tris-hydrochloric acid buffer solution of pH 7.5 in the case of polymer particles having amino groups, and an acetic acid buffer solution of pH 4.5 in the case of polymer particles having sulfonic acid groups), followed by pH adjustment with HCℓ or sodium acetate. Five mℓ of the polymer particles are sampled and subjected to centrifugal separation to remove the buffer solution. The polymer particles are then put into 200 mℓ of a 4 g/ℓ bovine serum albumin solution of 10 °C, and the mixture was maintained at 10 °C for 6 hours under gentle stirring. From the difference between the absorbance at 280 nm of the supernatant and the absorbance at 280 nm of the initial albumin solution, the amount of albumin adsorbed on the polymer particles is calculated.

Now, the present invention will be described in further detail with reference to Examples. However, it should be understood that the present invention is by no means restricted by such specific Examples.

EXAMPLE 1

95.7 g of glycidyl methacrylate, 4.3 g of diethylene glycol dimethacrylate and 1.0 g of 2,2-azobis(2,4-dimethylvaleronitrile) were dissolved in 87 g of toluene. This monomer solution was added to 600 g of an aqueous solution having 120 g of calcium chloride, 6 g of polyvinyl alcohol and 0.006 g of sodium nitrite dissolved in deionized water, followed by stirring to disperse the monomer solution in the aqueous solution. In this state, suspension polymerization was conducted at 70 °C for 6 hours to obtain white spherical crosslinked copolymer particles having a particle size of from about 30 to 300 µm (yield: 91%). The copolymer particles had an apparent volume of 2.7 mℓ/g in a dried state and 10.8 mℓ/g in a state swelled in 1,4-dioxane. Further, when the crosslinked copolymer particles were dried and then put in water, no substantial change was observed in the volume.

To 30 g of the crosslinked copolymer particles having glycidyl groups, 300 mℓ of 1,4-dioxane was added, and the mixture was maintained at room temperature for 3 hours to have the copolymer particles swelled with dioxane. Then, 150 mℓ of 1,4-dioxane and 150 mℓ of a 50 g/ℓ sulfuric acid aqueous solution were further added, and the mixture was heated to 50 °C and maintained at that temperature for 3 hours to hydrolyze the glycidyl groups to glycerol residues. The polymer particles having pendent glycerol residues thus obtained, had an apparent volume of 2.6 mℓ/g in a dried state and an apparent volume of 8.5 mℓ/g in a state swelled in water. Further, the cation exchange capacity was estimated to be about 0.05 meq/mℓ, and the exclusion limiting molecular weight was about 1,000,000.

EXAMPLE 2

The copolymerization reaction and the hydrolysis were conducted in the same manner as in Example 1 except that in Example 2, 91.3 g of glycidyl methacrylate and 8.7 g of diethylene glycol dimethacrylate were used respectively.

The resulting crosslinked copolymer having glycidyl groups had an apparent volume of 1.9 mℓ/g in a dried state and an apparent volume of 7.1 mℓ/g in a state swelled in 1,4-dioxane.

The polymer particles having pendent glycerol residues had an apparent volume of 7.5 mℓ/g in a state swelled in water. Further, the exclusion limiting molecular weight was about 500,000.

EXAMPLE 3

100 mℓ of the swelled polymer particles having pendent glycerol residues obtained in Example 1, were subjected to centrifugal separation to remove water. The polymer particles were then put in a solution mixture comprising 24 mℓ of a 5N NaOH aqueous solution and 40 mℓ of isopropanol, and the mixture was maintained at 50°C for one hour under gentle stirring. To this slurry containing the swelled polymer particles, a solution having 31 g of 2-chloroethyldiethylamine hydrochloride ($C\ell\text{-}CH_2\text{-}CH_2\text{-}N\text{-}(CH_2CH_3)_2{\bullet}HC\ell$) dissolved in 10 g of water, was added, and the mixture was maintained at 50°C for 5 hours.

White spherical anion exchange resin particles thus obtained were estimated to have an apparent volume of about 2.0 mℓ/g in a dried state and an apparent volume of about 8.5 mℓ/g in a state swelled in water. Further, the ion exchange capacity was 0.3 meq/mℓ. The absorption capacity for albumin was 58 g/ℓ.

EXAMPLE 4

About 10 g of the crosslinked copolymer having glycidyl groups obtained in the same manner as in Example 1 was put in 100 mℓ of a 1 mol/ℓ sodium sulfite aqueous solution, and the mixture was maintained at 50°C for 3 hours under stirring. The polymer particles were recovered by filtration and then put in 100 mℓ of tetrahydrofuran. The mixture was maintained at 25°C for 3 hours to let polymer particles swell. Further, 50 mℓ of tetrahydrofuran and 150 mℓ of a 50 g/ℓ sulfuric acid aqueous solution were added thereto, and the mixture was maintained at 50°C for 3 hours under stirring to hydrolyze glycidyl groups to glycerol residues.

Resulting white spherical particles had an apparent volume of 2.5 mℓ/g in a dried state and an apparent volume of about 10 mℓ/g in a state swelled in water. Further, the ion exchange capacity was 0.15 meq/mℓ. The absorption capacity for albumin was 85 g/ℓ.

EXAMPLE 5

Suspension polymerization was conducted in the same manner as in Example 1 except that in Example 2, 93.1 g of glycidyl methacrylate and 6.9 g of diethylene glycol dimethacrylate were used respectively. Resulting white spherical crosslinked copolymer particles had an apparent volume of 2.1 mℓ/g in a dried state and an apparent volume of 8.3 mℓ/g in a state swelled in 1,4-dioxane. Further, the particle size of the dried polymer was from about 30 to 250 µm.

To 30 g of the crosslinked copolymer particles, 300 mℓ of tetrahydrofuran was added, and the mixture was maintained at room temperature for 3 hours to let the particles swell. Then, 150 mℓ of tetrahydrofuran and 150 mℓ of a 50 g/ℓ sulfuric acid aqueous solution were further added, and the mixture was maintained at 50°C for 3 hours.

Polymer particles having pendent glycerol residues thereby obtained had an apparent volume of 2.4 mℓ/g in a dried state and an apparent volume of 7.7 mℓ/g in a state swelled with water. The exclusion limiting molecular weight was about 1,000,000.

EXAMPLE 6

About 100 mℓ of the swelled polymer particles having pendent glycerol residues obtained in Example 1, was subjected to centrifugal separation to remove water. The polymer particles were put in a solution mixture comprising 24 mℓ of a 10N NaOH aqueous solution and 40 mℓ of isopropanol, and the mixture was maintained at 50°C for one hour under gentle stirring. Then, to this slurry, 62 g of a 50% aqueous solution of 2-chloroethyldiethylamine hydrochloride was added, and the mixture was maintained at 50°C for 5 hours.

White spherical anion exchange resin particles thereby obtained had an apparent volume of 11 mℓ/g in a state swelled in water, and the anion exchange capacity was 0.24 meq/mℓ.

EXAMPLE 7

100 mℓ of the swelled polymer particles having pendent glycerol residues obtained in the same manner as in Example 1 were put in 24 mℓ of a 5N NaOH aqueous solution, and the mixture was maintained at 50°C for one hour under gentle stirring. To this slurry, a solution having 31 g of 2-chloroethyl-diethylamine hydrochloride dissolved in 10 g of water, was added, and the mixture was maintained at 50°C for 5 hours.

White spherical anion exchange resin particles thus obtained were estimated to have an apparent

volume of about 2 mℓ/g in a dried state and an apparent volume of about 5 mℓ/g in a state swelled in water, thus being inferior in the swellability to the product in Example 3. Further, the ion exchange capacity of this anion exchange resin was 0.3 meq/mℓ. The absorption capacity for albumin was 5 g/ℓ.

EXAMPLE 8

To 30 g of the crosslinked copolymer particles obtained in the same manner as in Example 1, 150 mℓ of a 100 g/ℓ sulfuric acid aqueous solution was added, and the mixture was maintained at 50 °C for 5 hours to hydrolyze the glycidyl groups to glycerol groups. White polymer particles having pendent glycerol residues thus obtained, had an apparent volume of 3.2 mℓ/g in a state swelled in water, thus being substantially inferior in the swellability to the product in Example 1.

## Claims

1. A crosslinked copolymer of glycidyl methacrylate with a crosslinking agent of the formula (1):

$$CH_2=C(CH_3)-COO{+}R-O{+}_nOC-C(CH_3)=CH_2 \qquad (1)$$

wherein R is a $C_{2-4}$ alkylene group, and n is an integer of from 1 to 4, which has an apparent volume in a dried state and in water of from 1.8 to 3.0 mℓ/g and an apparent volume in a state swelled with 1,4-dioxane of from 5.0 to 15.0 mℓ/g.

2. The crosslinked copolymer according to Claim 1, wherein the crosslinking agent is diethylene glycol dimethacrylate.

3. A method for polymerizing by suspension polymerization in an aqueous medium a polymerizable mixture consisting essentially of a monomer mixture comprising glycidyl methacrylate and a crosslinking agent of the formula (1):

$$CH_2=C(CH_3)-COO{+}R-O{+}_nOC-C(CH_3)=CH_2 \qquad (1)$$

wherein R is a $C_{2-4}$ alkylene group, and n is an integer of from 1 to 4, an organic solvent compatible with the monomer mixture, and a polymerization initiator, wherein the weight ratio of the glycidyl methacrylate and the crosslinking agent in the monomer mixture is selected within a range of from 97.5:2.5 to 90:10, and the volume ratio of the monomer mixture to the organic solvent is selected within a range of from 1:0.5 to 1:1.5, to form a crosslinked copolymer having an apparent volume in a dried state and in water of from 1.8 to 3.0 mℓ/g and an apparent volume in a state swelled with 1,4-dioxane of from 5.0 to 15.0 mℓ/g.

4. The method according to Claim 3, wherein the crosslinking agent is diethylene glycol dimethacrylate.

5. A hydrophilic separating agent which is a hydrolyzate of particles of a crosslinked copolymer of glycidyl methacrylate with a crosslinking agent of the formula (1):

$$CH_2=C(CH_3)-COO{+}R-O{+}_nOC-C(CH_3)=CH_2 \qquad (1)$$

wherein R is a $C_{2-4}$ alkylene group, and n is an integer of from 1 to 4, and which has an apparent volume in a dried state of from 2.0 to 3.0 mℓ/g and an apparent volume in a state swelled with water of from 6.0 to 13.0 mℓ/g.

6. The hydrophilic separating agent according to Claim 5, wherein the crosslinking agent is diethylene glycol dimethacrylate.

7. The hydrophilic separating agent according to Claim 5, which has a cation exchange capacity in a state

swelled with water of not higher than 0.05 meq/mℓ.

**8.** Hydrophilic crosslinked particles having pendent glycerol residues, which has an apparent volume in a dried state of from 2.0 to 3.0 mℓ/g and an apparent volume in a state swelled with water of from 6.0 to 13.0 mℓ/g.

**9.** A process for producing hydrophilic polymer particles capable of swelling in water to a volume of at least twice the volume in a dried state, which comprises permitting a crosslinked copolymer of glycidyl methacrylate with a crosslinking agent of the formula (1):

$$CH_2=C(CH_3)-COO(R-O)_nOC-C(CH_3)=CH_2 \qquad (1)$$

wherein R is a $C_{2-4}$ alkylene group, and n is an integer of from 1 to 4, and which has an apparent volume in a dried state of from 1.8 to 3.0 mℓ/g and an apparent volume in a state swelled with 1,4-dioxane of from 5.0 to 15.0 mℓ/g, to swell with a hydrophilic organic solvent, and then reacting the swelled crosslinked copolymer with water to hydrolyze glycidyl groups to glycerol groups.

**10.** The process according to Claim 9, wherein the resulting polymer particles have an apparent volume in a dried state of from 2.0 to 3.0 mℓ/g and an apparent volume in a state swelled with water of from 6.0 to 13.0 mℓ/g.

**11.** The process according to Claim 9, wherein the crosslinked copolymer is reacted with water in a state where the crosslinked copolymer is swelled with the organic solvent to a volume of at least twice the volume in a dried state.

**12.** A hydrophilic ion exchange resin having pendent glycerol residues and amino groups or sulfonic acid groups, which has an apparent volume in a dried state of from 2.0 to 3.0 mℓ/g and an apparent volume in a state swelled with water of from 6.0 to 13.0 mℓ/g.

**13.** The ion exchange resin according to Claim 12, which has an ion exchange capacity of from 0.1 to 0.3 meq/mℓ per 1 mℓ of the particles in a state swelled with water.

**14.** A process for producing a basic anion exchange resin capable of swelling in water to a volume of at least twice the volume in a dried state, which comprises treating hydrophilic crosslinked particles having pendent glycerol residues, which have an apparent volume in a dried state of from 2.0 to 3.0 mℓ/g and an apparent volume in a state swelled with water of from 6.0 to 13.0 mℓ/g, with a solution mixture comprising an alkali metal hydroxide, water and a hydrophilic organic solvent to obtain swelled particles having the solution mixture penetrated to the interior, and reacting to the swelled particles a haloalkylamine of the formula (2):

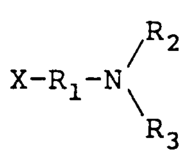

(2)

wherein X is a halogen atom, $R_1$ is an alkylene group, and each of $R_2$ and $R_3$ is an alkyl group or a hydroxyalkyl group, or a salt thereof.

**15.** The process according to Claim 14, wherein the hydrophilic crosslinked particles are treated with the solution mixture to obtain crosslinked particles having the solution mixture penetrated to the interior and swelled to a volume of at least twice the volume in the dried state, and the reaction with the haloalkylamine or its salt is conducted while substantially maintaining this swelled state.

**16.** The process according to Claim 14, wherein the resulting basic anion exchange resin has an apparent volume in a dried state of from 2.0 to 3.0 mℓ/g, an apparent volume in a state swelled with water of from 6.0 to 13.0 mℓ/g and an ion exchange capacity of from 0.1 to 0.3 meq/mℓ.

**17.** A process for producing a water-swellable acidic cation exchange resin having both pendent glycerol residues and sulfonic acid groups, which comprises reacting an aqueous solution containing at least one member selected from the group consisting of sulfites and hydrogensulfites to a crosslinked copolymer of glycidyl methacrylate with a crosslinking agent of the formula (1):

$$CH_2=C(CH_3)-COO\text{+}R-O\text{)}_nOC-C(CH_3)=CH_2 \qquad (1)$$

wherein R is a $C_{2-4}$ alkylene group, and n is an integer of from 1 to 4, which has an apparent volume in a dried state of from 1.8 to 3.0 mℓ/g and an apparent volume in a state swelled with 1,4-dioxane of from 5.0 to 15.0 mℓ/g, then treating the copolymer with a hydrophilic organic solvent to have the organic solvent penetrated to the interior and to have the copolymer swelled to a volume of at least twice the volume in the dried state, and then reacting water to the swelled copolymer to hydrolyze the residual glycidyl groups to glycerol groups.

**18.** A method for recovering a protein, which comprises passing an aqueous solution containing a protein through a column packed with a hydrophilic ion exchange resin having pendent glycerol residues and amino groups or sulfonic acid groups, which has an apparent volume in a dried state of from 2.0 to 3.0 mℓ/g, an apparent volume in a state swelled with water of from 6.0 to 13.0 mℓ/g and an ion exchange capacity of not higher than 0.4 meq/mℓ, to have the protein adsorbed in an amount of at least 30 g per liter of the resin, and then passing an eluent through the column to elute the adsorbed protein.

European Patent Office

# EUROPEAN SEARCH REPORT

Application Number

EP 91111963.4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP - A2 - 0 320 023 (CESKOSLOVENSKA AKADEMIE VED; AKADEMIA NAUK SSSR) * Claims 1,2,5-7,9,11-18,22; examples 11-18 * | 1,5,9, 11,12, 17,18 | C 08 F 220/32 //(C 08 F 220/32 C 08 F 220:18) C 08 F 2/18 C 08 F 8/00 B 01 J 20/26 B 01 J 39/20 B 01 J 41/14 B 01 D 15/08 |
| X | EP - A2 - 0 154 343 (RESEARCH DEVELOPMENT CORPORATION OF JAPAN) * Claims 1-5, 14,15; page 14, lines 19-27; page 16, lines 23-25; example 6 * | 1,2,5, 9,11, 12,18 | |
| P,X | PATENT ABSTRACTS OF JAPAN, unexamined applications, C section, vol. 14, no. 334, July 18, 1990 THE PATENT OFFICE JAPANESE GOVERNMENT page 112 C 742 * Kokai-no. 2-123 105 (MITSUBISHI KASEI CORP.) 10-05-1990 * | 1,5, 12,14, 15 | **TECHNICAL FIELDS SEARCHED (Int. Cl.5)** |
| X | CHEMICAL ABSTRACTS, vol. 92, no. 8, February 25, 1980, Columbus, Ohio, USA JOSHIDA et al. "Controlled release of potassium chloride from radiation-polymerized copolymer matrices" pages 380-381, abstract-no. 64 682f & JAKUZAIGAKU 1979, 39(2), 69-74 * | 1,2,4-6 | A 61 K 37/00 B 01 D 15/00 B 01 D 71/00 B 01 J 20/00 B 01 J 39/00 B 01 J 41/00 C 08 F 2/00 C 08 F 8/00 C 08 F 20/00 C 08 F 220/00 |
| X | CHEMICAL ABSTRACTS, vol. 99, no. 1, July 4, 1983, Columbus, Ohio, USA MITSUBISHI CHEMICAL INDU- | 1,3,5, 9,12, 13,16 | |

The present search report has been drawn up for all claims

| Place of search | Date of completion of the search | Examiner |
|---|---|---|
| VIENNA | 08-10-1991 | PUSTERER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application Number

-2-

EP 91111963.4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| | STRIES CO. LTD. "Hydrophilic weakly acidic cation-exchange resin" page 41, abstract-no. 6 659n & JP-A2-58-17 844 -- | | |
| X | CHEMICAL ABSTRACTS, vol. 99, no. 6, August 8, 1983, Columbus, Ohio, USA MITSUBISHI CHEMICAL INDUSTRIES CO. LTD. "Hydrophilic weakly basic anion-exchange resin" page 33, abstract-no. 39 282n & JP-A2-58-24 354 -- | 1,3,5, 9,12, 13,16 | |
| X | CHEMICAL ABSTRACTS, vol. 104, no. 5, February 3, 1986, Columbus, Ohio, USA YOSHIKAWA et al. "Isolation and purification of proteins" page 295, abstract-no. 31 355q & JP-A2-60-169 427 (MITSUBISHI CHEMICAL INDUSTRIES CO.LTD.) -- | 1,5,9, 12,18 | TECHNICAL FIELDS SEARCHED (Int. Cl.5) |
| X | CHEMICAL ABSTRACTS, vol. 95, no. 1, July 6, 1981, Columbus, Ohio, USA SVEC et al. "Immobilization of ampholitic compounds on a insoluble carrier" page 279, abstract-no. 2 666g & CS-B-185 717 -- | 1,5,9, 12,18 | |
| X | US - A - 4 794 152 | 1,3,5, | |

The present search report has been drawn up for all claims

| Place of search | Date of completion of the search | Examiner |
|---|---|---|
| VIENNA | 08-10-1991 | PUSTERER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application Number



EP 91111963.4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| | (KABASHI et al.) * Claims 1,4,5; examples 2-4 * -- | 9 | |
| X | CHEMICAL ABSTRACTS, vol. 112, no. 16, April 16, 1990, Columbus, Ohio, USA JELINKOVA et al. "Reactive polymers. 58. Polyampholytes based on macroporous co-polymers of glycidyl metha-crylate with ethylene glycol dimethacrylate of divinyl-benzene." page 65, abstract-no. 140 724v & REACT. POLYM. 1989, 11(3), 253-60 -- | 1,5,12 | |
| X | CHEMICAL ABSTRACTS, vol. 87, no. 6, August 8, 1977, Columbus, Ohio, USA SVEC et al. "Reaction of a macroporous copolymer glycidyl methacrylate-ethylene dimetha-crylate with sodium sulfide and the use of the product as a catalyst." page 14, abstract-no. 40 017q & CHEM. IND. 1977, (4), 159 ---- | 1,9, 12,17 | TECHNICAL FIELDS SEARCHED (Int. Cl.5) |

The present search report has been drawn up for all claims

| Place of search | Date of completion of the search | Examiner |
|---|---|---|
| VIENNA | 08-10-1991 | PUSTERER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document